# EUROPEAN PATENT APPLICATION

(11) **EP 2 065 054 A1**
(43) Date of publication of application: **03.06.2009**
(21) Application number: 07076033.5
(22) Date of filing: 29.11.2007
(51) Int. Cl.: A61K 45/06, A61P 35/00

(54) **Combinations comprising a prostaglandin and uses thereof**

(71) Applicant: Bayer Schering Pharma Aktiengesellschaft, 13353 Berlin (DE)
(72) Inventor: Hoffmann, Jens, 16567 Berlin (DE); Klar, Ulrich, 13503 Berlin (DE); Skuballa, Werner, 13465 Berlin (DE)

(57) **Abstract**

The invention relates to a combination comprising (a) a compound selected from the class of prostaglandins and (b) a compound selected from the class of tubulin/microtubule interfering agents and/or (c) a compound selected from the class of cyclooxygenase inhibitors.

## Description

The invention relates to a combination comprising (a) a compound selected from the class of prostaglandins and (b) a compound selected from the class of tubulin/microtubule interfering agent and a combination comprising (a) a compound selected from the class of prostaglandins and (b) a compound selected from the class of tubulin/microtubule interfering agent and (c) a compound selected from the class of cyclooxygenase inhibitors .

The invention further relates to a method for the therapy of solid tumors and other tumor diseases with said combinations and the use of the combinations for the treatment of solid tumors and other tumor diseases.

One aspect of the invention relates to a method for treating lung cancer with the combinations of the invention.

Another aspect of the invention relates to a method for treating non-small-cell lung cancer with the combinations of the invention.

The invention further relates to a pharmaceutical composition of a combination comprising (a) a prostaglandin, (b) a tubulin/microtubule interfering agent and a combination comprising (a) a prostaglandin, (b) a tubulin/microtubule interfering agent and (c) a cyclooxygenase inhibitor and a pharmaceutically acceptable carrier.

Furthermore the present invention relates to a kit comprising (a) a prostaglandin, (b) a tubulin/microtubule interfering agent and a kit comprising (a) a prostaglandin, (b) a tubulin/microtubule interfering agent and (c) a cyclooxygenase inhibitor wherein optionally some components or all of them are in the form of a pharmaceutical formulation ready for use to be administered simultaneously, concurrently, separately or sequentially. The drugs may be administered independently of one another by oral, intravenious, topical, local installations, intraperitoneal or nasal application routes.

### Background of the invention

During the last decades the use of cytotoxic compounds has become a cornerstone in the treatment of various solid tumors in first, second and third line settings as single agents or in combinations. Although the increased number of treatment options led to some increase of the survival time, the therapies are still connected with considerable side effects which often prevents the application of sufficient doses and/or number of treatment cycles.

Therefore, there is still a tremendous need to improve the therapeutic window of chemotherapies.

It was thus the object of the present invention to provide a combination which showed superior effects over the components administered alone.

It is known in the art that during neovascularisation in endothelial cells as well as in epithelial cancer cells being present in human lung-, breast-, colon- and prostate cancer cells cyclooxygenease-2 (COX-2) is expressed. Cyclooxygenase-2 however induces PGE₂ or PGl₂ synthesis. The prostaglandins mentioned accelerate angiogenesis via VEGF induction.

It is further postulated in WO 03/035047 disclosing a combination of a specific COX-2 inhibitor and a VEGF-inhibitor that said combination is suitable for treatment of colon or prostate cancer due to the fact that the COX-2 inhibitor/VEGF inhibitor combination has effects on neovascularisation by reducing the number of VEGF receptors and at the same time inhibiting the VEGF receptors present.

Prostaglandins (PG) are known in the art, such as e.g. PGl₂, PGE₁, 6-oxo-PGE₁, PGE₂ as well as their more stable derivatives, such as e.g. Iloprost, Cicaprost, Misoprostol. PGE₂ and PGl₂ induce VEGF receptors in endothelial cells and are thus involved in acceleration of angiogenesis.

From these facts it seems to be rather unfavourable to add prostaglandins to a composition intented for use in antitumor therapy.

It is thus more than surprising that combining a prostaglandin and a tubulin/microtubule interfering agent results in a combination being well suitable for the treatment of solid tumors.

### Detailed description of the invention

One aspect of the present invention therefore relates to a combination of (a) a compound selected from the class of tubulin/microtubule interfering agents and (b) a compound selected from the class of prostaglandins.

A further surprising effect found in the present invention is that adding to the mentioned combination comprising and (a) a prostaglandin or any of its derivatives and (b) a tubulin/microtubule interfering agent a further component (c) a compound selected from the class of cyclooxygenase inhibitors also results in a combination having synergistic effects.

Thus another aspect of the present invention relates to a combination of (a) compound selected from the class of prostaglandins and (b) a compound selected from the class of tubulin/microtubule interfering agents and (c) a compound selected from the class of cyclooxygenase inhibitors.

Another aspect of the invention relates to a combination of (a) compound selected from the class of prostaglandins and (b) a compound selected from the class of epothilones and/or (c) a compound selected from the class of cyclooxygenase inhibitors.

The present invention further relates to a combination comprising (a) a prostaglandin which is preferentially 6-Oxo-PGE₁, PGE₂, PGl₂ or TXA₂-antagonist or any of their analogs or derivatives and (b) a microtubule stabilizing/interfering agent and/or (c) a cyclooxygenase inhibitor, being a non-steroidal inflammation inhibitor with cyclooxygenase inhibiting activity.

The naturally occurring prostaglandins are often not stable enough for administration and are to be derivatized to be useful for the treatment of animals or humans. The different classes of prostaglandin compounds and their suitabilities are well known in the art. For the use in the present invention PGE₁, 6-Oxo-PGE₁, PGE₂, PGl₂ and their analogs and derivatives are preferred , especially the chemically and metabolically more stable prostacycline (PGl₂) derivatives, the carbacyclines, Eptaloprost, Beraprost, especially Iloprost (ILOMEDIN^{™}, VENTAVIS^{™}). and Cicaprost, a stable carbacyclin derivative (X= -CH₂- in formula II described below), being disclosed in the same patent as Iloprost, EP 11 591 and US 4,692,464 which are incorporated by reference herein.

As microtubule interfering agents the following compounds selected from the structural classes of taxanes, vinca alkaloids, podophyllotoxins, discodermolides, cryptophycins, tubulysins, laulimalides, dictyostatins and epothilones can be used.
■ Taxanes are known to be useful for the treamtent of cancer, especially paclitaxel and docetaxel.
■ Vinca alkaloids are broadly used for the treatment of cancer. Especially mentioned are vincristine (FARMISTIN^{™}), vinblastine (VINBLASTIN^{™}), vinorelbine as well as their pharmaceutically acceptable salts.
■ Podophyllotoxins are also marketed, such as etoposide (ETOPOPHOS^{™}) and teniposide (VM 26-BRISTOL^{™}).
■ Discodermolides are polyketide anticancer drug compounds whose synthesis is disclosed in e.g. WO 98/24429.
■ Epothilones represent a novel class of tubulin/microtubule interfering agents. Different publications and reports on study results (e.g. IDrugs, 2002, 5(10):949-954) have proven their anticancer activity. Dose regimens are known from the study reports or from other publications e.g. for epothilones A and B from WO 99/43320.

Epothilone derivatives are known from the art e.g. from WO 93/10102, WO 93/10121 and DE 41 38 042 A2, WO 97/19086 and WO 98/25929 , WO 99/43320, WO 2000/066589, WO 00/49021 WO 00/71521 , WO 2001027308 , WO 99/02514 , WO 2002080846.

The present invention further relates to a combination of (a) a prostaglandin, preferetially selected from the prostaglandin subtypes PGE₁, 6-Oxo-PGE₁, PGl₂, preferably PGl₂ and any of their analogs or derivatives, (b) one tubulin/microtubule interfering agent such as e.g. taxanes, vinca alkaloids, podophyllotoxins, discodermolides, cryptophycins, tubulysins, laulimalides, dictyostatins, epothilones and/or (c) a cyclooxygenase inhibitor or all non-steroidal inflammation inhibitors with cyclooxygenase inhibiting activity e.g. Diclofenac (Voltaren^{™}), Indometacin (Amuno^{™}), lbuprofen (Brufen^{™}), Ketoprofen, Naproxen, O-acetyl salicylic acid, Celecoxib, Refecoxib preferably O-acetylsalicylic acid (ASPIRIN^{™}) as well as any derivatives, prodrugs, pharmaceutically acceptable salts and crystal modifications such as e.g. solvates, polymorphs thereof.

One aspect of the invention are combinations of (a) a prostaglandin and (b) a tubulin/microtubule interfering agent and any of the currently marketed products containing O-acetylsalicylic acid or any of its combinations

A combination of acetylsalicylic acid on the market suitable for the present combination is e.g. the combination of acetylsalicylic acid with vitamin C.

The target cyclooxygenase is involved in the natural synthesis of prostaglandins. Cyclooxygenase is known to be constituted of two different isoforms, a constitutive isoform, designated as cyclooxygensase-1 (COX-1) and an inducible isoform designated as cyclooxygenase-2 (COX-2). Cyclooxygenase inhibitors are known from the art and marketed for a long time e.g. such as Diclofenac (Voltaren^{™}), Indometacin (Amuno^{™}), Ibuprofen (Brufen^{™}), Ketoprofen, Naproxen, Aspirin^{™}, Celecoxib, Refecoxib .

It has now surprisingly been found that if a prostaglandin is added to the combination of an epothilone and a COX inhibitor a reduced toxic effect can be observed while the desired antitumor effects are retained.

In one aspect of the invention the prostaglandin is selected from the group of PGE₁ and PGE₁ derivatives of the compounds represented in the following formula (I) 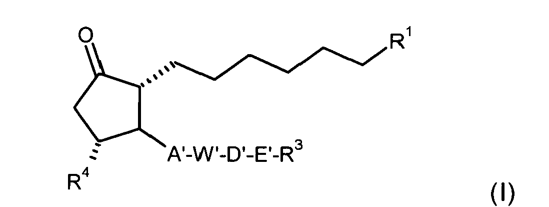 or the 6-oxo-PGE₁ derivatives of the formula (II) 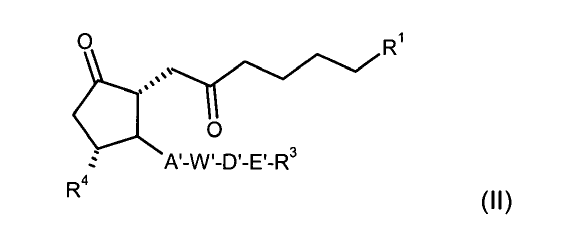 wherein
R¹ is COOR²
   whereby R² means hydrogen, a C₁-C₁₀-alkyl group, a C₅-C₆-cycloalkyl group, or a C₆-C₁₀-aryl group or a 5- or six-membered heterocyclic ring or a C(O)NHSO₂-R⁵ group
   whereby R⁵ is C₁-C₁₀-alkyl, C₅-C₆-cycloalkyl or C₆-C₁₀ aryl,
A' is a CH=CH-group having E-configuration or a -C≡C- group,
W' is a free or protected hydroxymethylen group or a free or protected 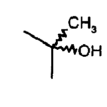 group,
   whereby the hydroxyl group may be in α- or β-position,
D'is a straight or branched C₁-C₅ alkylen group
E' is a -C≡C- group or a C₂-C₄-alkenylen group,
R³ is a C₁-C₁₀-alkyl group, a C₃-C₁₀-cydoalkyl group or an optionally substituted aryl group or a 5- or six-membered heterocyclic ring,
R⁴ is a free or protected hydroxy group
and if R² is hydrogen, its salts with physiologically suitable bases as well as α-, β-, or γ-Cyciodextrinclathrates of thereof
or
the fluoro-PGE₁ derivatives of formula III 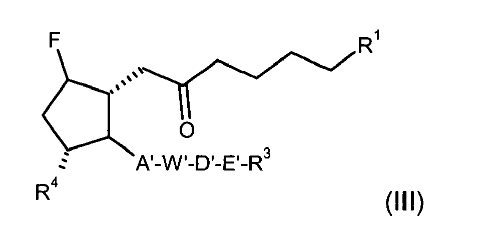 wherein
R¹ is COOR² or C(O)NHSO₂R²
   whereby R² means a C₁-C₁₀-alkyl group, a C₅-C₆-cycloalkyl
   group, or a C₆-C₁₂-aryl group or a 5- or six-membered heterocyclic ring and if R² is COOR², R² means additionally hydrogen or a phenacyl group which is optionally substituted by 1-3 halogen atoms,
A' is a CH=CH-group having E-configuration or a -C≡C- group,
W' is a free or protected hydroxymethylen group or a free or protected 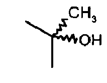 group,
   whereby the hydroxyl group may be in α- or β-position,
D'is a straight or branched C₁-C₅ alkylen group or a direct bond
E' is a -C≡C- group or a C₂-C₄-alkenylen group or a group 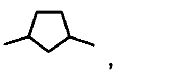
R³ is a hydrogen atom, a C₁-C₁₀-alkyl group, a C₁-C₁₀-alkenyl group, a C₃-C₁₀-cycloalkyl group or an optionally substituted aryl group or a 5- or six-membered heterocyclic ring,
R⁴ is a hydrogen atom, a methyl group, a free or protected hydroxy group and if R² is hydrogen, its salts with physiologically suitable bases as well as α-,
β-, or γ-Cyclodextrinclathrates of thereof
or
9-Oxo-11 α, 16-dihydroxy-16-methyl-prost-13(E)-en-1-oic acid

In one aspect of the invention the prostaglandin or prostaglandin derivative (a) of the combination as claimed in claim 1 or 2 is a prostacyclin or carbacyclin derivative or analog.

In one aspect of the invention the prostaglandin is selected from the group of prostacyclin derivatives of formula (IV) 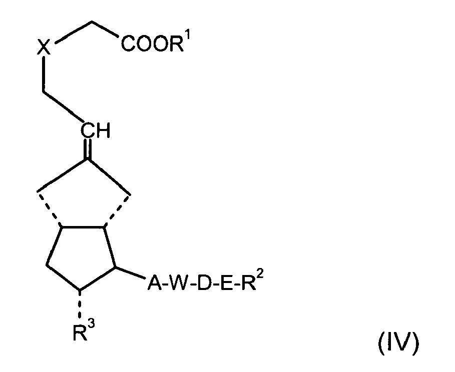 wherein
R¹ is hydrogen,
   a C₁-C₁₀-alkyl group being optionally substituted by halogen, phenyl, dimethylamino, diethylamino, C₁-C₅-alkoxy,
   a C₅-C₆-cycloalkyl group,
   a phenyl group being optionally substituted by halogen, C₁-C₄-alkoxy, hydroxyl or trifluoromethyl,
   or a 5 or 6 membered heterocyclic ring having at least one nitrogen, oxygen or sulphur atom,
A is a group selected from -CH₂-CH₂-, trans -CH=CH- or -C≡C-,
W is a hydroxy group which optionally may be etherified or esterified or a group 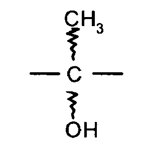
wherein the hydroxy group may optionally be etherified or esterified and the hydroxyl group may be in α- or β-position
D and E together form an additional bond or
D is a straight or branched C₁-C₁₀- alkylen group or an unsaturated
C₂-C₁₀-alkenylene group which optionally may be substituted by fluorine atoms,
E is an oxygen atom or the group -C≡C-, or a direct bond and
R² is a C₁-C₇ alkyl group or a C₅-C₆-cycloalkyl group which both optionally may be substituted by
   halogen, an optionally substituted phenyl group, a C₅-C₆-cycloalkyl group, C₁-C₄-alkyl, chloromethyl, or a 5 or 6-membered heterocyclic ring having at least one nitrogen, oxygen or sulphur atom,
R³ is a hydroxyl group which optionally can be etherified or esterified and
X is a group selected from -CH₂- or -O-.

One aspect of the invention are combinations comprising prostacyclines which in the sense of the present invention are compounds of formula IV above wherein X is oxygen.
Another aspect of the invention are combinations comprising carbacyclines which in the sense of the present invention are compounds of formula IV wherein X is a methylen group -CH₂-.

In yet another aspect of the invention the prostaglandin is a compound of formula IV wherein R² is a C₁-C₇ alkyl group which optionally may be substituted by halogen, an optionally substituted phenyl group, a C₅-C₆-cycloalkyl group, C₁-C₄-alkyl, chloromethyl, or a 5 or 6-membered heterocyclic ring having at least one nitrogen, oxygen or sulphur atom.

In another aspect of the invention the prostaglandin used for the combination of the present invention is selected from the group consisting of 6-Oxo-PGE₁ derivatives
(13E)-(11R, 15S, 16RS)-6,9-Dioxo-11,15-dihydroxy-16-methyl-18,18,19,19-tetradehydro-13-prostenoic acid,
(13E)-(11R, 15S, 16RS)-6,9-Dioxo-11,15-dihydroxy, 16-methyl-18,18,19,19-tetradehydro-13-prostenoic acid methylester,
(11 R, 15S, 16S)-6,9-Dioxo-16,20-dimethyl-13,14,18,18,19,19-hexadehydro-13-prostenoic acid
(11R, 15S, 16S)-6,9-Dioxo-16,20-dimethyl-13,14,18,18,19,19-hexadehydro-13-prostenoic acid methylester, (9R, 11 R, 13E, 15S, 16S)-6-Oxo-9-fluoro-11,15-dihydroxy-16-methyl-18,18,19,19-tetradehydro-13-prostenoic acid, (9R, 11R, 13E, 15S, 16S)-6-Oxo-9-fluoro-11,15-dihydroxy-16-methyl-18,18,19,19-tetradehydro-13-prostenoic acid methylester,
(9S, 11R, 13E, 15S, 16S)-6-Oxo-9-fluoro-11,15-dihydroxy-16-methyl-18,18,19,19-tetradehydro-13-prostenoic acid,
(9S, 11R, 13E, 15S, 16S)-6-Oxo-9-fluoro-11,15-dihydroxy-16-methyl-18,18,19,19-tetradehydro-13-prostenoic acid methylester,
(9R, 11R, 15S, 16S)-6-Oxo-9-fluoro-11,15-dihydroxy-16,20-dimethyl-13,14,18,18,19,19-hexadehydro-13-prostenioc acid,
(9S, 11R, 15S, 16S)-6-Oxo-9-fluoro-11,15-dihydroxy-16,20-dimethyl-13,14,18,18,19,19-hexadehydro-13-prostenioc acid,
(9R, 11R, 15S, 16S)-6-Oxo-9-fluoro-11,15-dihydroxy-16,20-dimethyl-13,14,18,18,19,19-hexadehydro-13-prostenioc acid methylester,
(9S, 11R, 15S, 16S)-6-Oxo-9-fluoro-11,15-dihydroxy-16,20-dimethyl-13,14,18,18,19,19-hexadehydro-13-prostenioc acid methylester,

In yet another aspect of the invention the prostaglandin suitable for the combination of the present invention is selected from the group consisting of PGE₁ derivatives
(13E)-(11R, 15S, 16RS)- 9-Oxo-11,15-dihydroxy-16-methyl-18,18,19,19-tetradehydro-13-prostenoic acid
(13E)-(11R, 15S, 16RS)-9-Oxo-11,15-dihydroxy, 16-methyl-18,18,19,19-tetradehydro-13-prostenoic acid methylester,
(11R, 15S, 16S)-9-Oxo-16,20-dimethyl-13,14,18,18,19,19-hexadehydro-13-prostenoic acid
(11R, 15S, 16S)-9-Oxo-16,20-dimethyl-13,14,18,18,19,19-hexadehydro-13-prostenoic acid methylester,(9R, 11R, 13E, 15S, 16S)-11,15-Dihydroxy-9-fluoro-16-methyl-18,18,19,19-tetradehydro-13-prostenoic acid, (9R, 11R, 13E, 15S, 16S)-11,15-Dihydroxy-9-fluoro-16-methyl-18,18,19,19-tetradehydro-13-prostenoic acid methylester,
(9S, 11R, 13E, 15S, 16S)-11,15-Dihydroxy-9-fluoro-16-methyl-18,18,19,19-tetradehydro-13-prostenoic acid,
(9S, 11R, 13E, 15S, 16S)-11,15-dihydroxy-9-fluoro-16-methyl-18,18,19,19-tetradehydro-13-prostenoic acid methylester
(9R, 11R, 15S, 16S)-11,15-Dihydroxy-9-fluoro-16,20-dimethyl-13,14,18,18,19,19-hexadehydro-13-prostenioc acid,
(9S, 11R, 15S, 16S)-11,15-dihydroxy-9-fluoro-16,20-dimethyl-13,14,18,18,19,19-hexadehydro-13-prostenioc acid,
(9R, 11R, 15S, 16S)-11,15-Dihydroxy-9-fluoro-16,20-dimethyl-13,14,18,18,19,19-hexadehydro-13-prostenioc acid methylester,
(9S, 11R, 15S, 16S)-11,15-Dihydroxy-9-fluoro-16,20-dimethyl-13,14,18,18,19,19-hexadehydro-13-prostenioc acid methylester,

One aspect of the invention is a combination wherein the prostaglandin is selected from the group consisting of ((E)-5-{(3aS,4R,5R,6aS)-5-Hydroxy-4-[(E)-(3S,4RS)-3-hydroxy)-4-methyl-1-octen-6-ynyl]perhydropentalen-2-ylidene}valeric acid and (5-{(E)-(1S,5S,6S,7R)-7-Hydroxy-6-[(3S,4S)-3-hydroxy-4-methyl-nona-1,6-diynyl]-bicyclo[3.3.0]oct-3-ylidene}-3-oxapentanoic acid)or (9-Oxo-11a. 16-dihydroxy-16-methyl-prost-13(E)-en-1-oic acid) (Misoprostol).

Especially preferred in the sense of the present invention are Iloprost ((E)-5-{(3aS,4R,5R,6aS)-5-Hydroxy-4-[(E)-(3S,4RS)-3-hydroxy)-4-methyl-1-octen-6-ynyl]perhydropentalen-2-ylidene}valeric acid and Cicaprost (5-{(E)-(1 S,5S,6S,7R)-7-Hydroxy-6-[(3S,4S)-3-hydroxy-4-methyl-nona-1,6-diynyl]-bicyclo[3.3.0]oct-3-ylidene}-3-oxapentanoic acid).

The epothilones as disclosed in the art e.g. WO 93/10102, WO 2000/066589, WO 2001027308 , WO 99/02514 , WO 2002080846are suitable for use in the present invention.

The epothilones and their derivatives as being suitable for the present invention as well as the production thereof is disclosed, for example in DE 19907588, WO 98/25929, WO 99/58534, WO 99/2514, WO 99/67252, WO 99/67253, WO 99/7692, EP 99/4915, WO 00/1333, WO 00/66589, WO 00/49019, WO 00/49020, WO 00/49021, WO 00/71521, WO 00/37473, WO 00/57874, WO 01/92255, WO 01/81342, WO 01/73103, WO 01/64650, WO 01/70716, US 6204388, US 6387927, US 6380394, US 02/52028, US 02/58286, US 02/62030, WO 02/32844, WO 02/30356, WO 02/32844, WO 02/14323, and WO 02/8440. Particularly interesting epothilones of the present invention may be produced according to WO 00/66589.

One aspect of the present invention are combinations according to the claims wherein the tubulin/microtubule interfering agent is paclitaxel, docetaxel or an epothilone.

One aspect of the present invention are combinations wherein the tubulin/microtubule interfering agents are epothilones as described by formula (V) 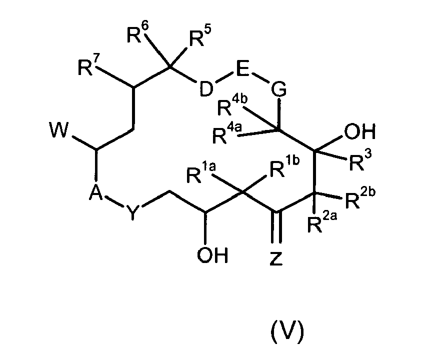 wherein:
- R^{1a}, R^{1b}: are each independently hydrogen, C₁-C₁₀ alkyl, aryl, aralkyl, or together form a -(CH₂)ₘ-group where m is 2 to 5;
- R^{2a}, R^{2b}: are each independently hydrogen, C₁-C₁₀ alkyl, aryl, aralkyl, or together form a -(CH₂)ₙ-group where n is 2 to 5, or are C₂-C₁₀ alkenyl or C₂-C₁₀ alkynyl;
- R³: is hydrogen, C₁-C₁₀ alkyl, aryl, aralkyl;
- R^{4a}, R^{4b}: are each independently hydrogen, C₁-C₁₀ alkyl, aryl, aralkyl, or together form a -(CH₂)ₚ- group where p is 2 to 5;
- R⁵: is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂N(alkyl, acyl)_{1,2}, or CH₂Hal, CHal₃;
- R⁶, R⁷: are each hydrogen, or together form an additional bond, or together form an epoxy function;
- G: is O or CH₂;
- D-E: together is a group -H₂C-CH₂-, -HC=CH-, -C≡C-, -CH(OH)-CH(OH)-, -CH(OH)-CH₂-, -CH₂-CH(OH)-, -CH₂-O-, -O-CH2- or CH-CH
whereby if G is O then D-E cannot be CH₂-O; or
D-E-G together is a group H₂C-CH=CH
- W: is a group C(=X)R⁸, or is an aromatic or heteroaromatic radical;
X is O or a group CR⁹R¹⁰;
- R⁸: is hydrogen, C₁-C₁₀ alkyl, aryl, aralkyl, halogen, CN;
- R⁹, R¹⁰: are each independently hydrogen, C₁-C₂₀ alkyl, aryl, aralkyl, or together with the methylene carbon form a 5- to 7-membered carbocyclic ring;
- Z: is O or H and the group OR¹¹;
- R¹¹: is hydrogen or a protecting group PG^{z};
- A-Y: is a group O-C(=O), O-CH₂, CH₂-C(=O), NR¹²-C(=O), NR¹²-SO₂;
- R¹²: is hydrogen, or C₁-C₁₀ alkyl;
- PG^{z}: is C₁-C₂₀ alkyl, C₄-C₇ cycloalkyl, which may contain one or more oxygen atoms in the ring, aryl, aralkyl, C₁-C₂₀ acyl, aroyl, C₁-C₂₀ alkylsulfonyl, arylsulfonyl, tri(C₁-C₂₀ alkyl)silyl, di(C₁-C₂₀ alkyl) arylsilyl, (C₁-C₂₀ alkyl)diarylsilyl, or tri(aralkyl)silyl;
as a single stereoisomer or a mixture of different stereoisomers,and / or as a pharmaceutically acceptable salt thereof.

Another embodiment for the combination of the invention are epothilones of formula (V)
wherein:
- R^{1a}, R^{1b}: are each independently hydrogen, C₁-C₁₀ alkyl, aryl, aralkyl, or together form a -(CH₂)ₘ-group where m is 2 to 5;
- R^{2a}, R^{2b}: are each independently hydrogen, C₁-C₁₀ alkyl, aryl, aralkyl, or together form a -(CH₂)ₙ-group where n is 2 to 5, or are C₂-C₁₀ alkenyl or C₂-C₁₀ alkynyl;
- R³: is hydrogen, C₁-C₁₀ alkyl, aryl, aralkyl;
- R^{4a}, R^{4b}: are each independently hydrogen, C₁-C₁₀ alkyl, aryl, aralkyl, or
- R⁵: together form a -(CH₂)ₚ- group where p is 2 to 5; is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂N(alkyl, acyl)_{1,2}, or CH₂Hal, CHal₃;
- R⁶, R⁷: are each hydrogen, or together form an additional bond, or together form an epoxy function;
- G: is O or CH₂;
- D-E: together is a group -H₂C-CH₂-, -HC=CH-, -C≡C-, -CH(OH)-CH(OH)-, -CH(OH)-CH₂-, -CH₂-CH(OH)-, -CH₂-O-, -O-CH₂- or 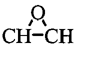 whereby if G is O then D-E cannot be CH₂-O; or
D-E-G together is a group H₂C-CH=CH
- W: is a group C(=X)R⁸, or is a bi- or tricyclic aromatic or heteroaromatic radical;
X is O or a group CR⁹R¹⁰;
- R⁸: is hydrogen, C₁-C₁₀ alkyl, aryl, aralkyl, halogen, CN; R⁹, R¹⁰ are each independently hydrogen, C₁-C₂₀ alkyl, aryl, aralkyl, or together with the methylene carbon form a 5- to 7-membered carbocyclic ring;
- Z: is O or H and the group OR¹¹;
- R¹¹: is hydrogen or a protecting group PG^{z};
- A-Y: is a group O-C(=O), O-CH₂, CH₂-C(=O), NR¹²-C(=O), NR¹²-SO₂;
- R¹²: is hydrogen, or C₁-C₁₀ alkyl;
- PG^{z}: is C₁-C₂₀ alkyl, C₄-C₇ cycloalkyl, which may contain one or more oxygen atoms in the ring, aryl, aralkyl, C₁-C₂₀ acyl, aroyl, C₁-C₂₀ alkylsulfonyl, arylsulfonyl, tri(C₁-C₂₀ alkyl)silyl, di(C₁-C₂₀ alkyl) arylsilyl, (C₁-C₂₀ alkyl)diarylsilyl, or tri(aralkyl)silyl;
as a single stereoisomer or a mixture of different stereoisomers,and / or as a pharmaceutically acceptable salt thereof.

Another embodiment for the combination of the invention are epothilones of formula (V)
wherein
- R^{1a}, R^{1b}: are each independently hydrogen, C₁-C₄ alkyl, or together form a-(CH₂)ₘ-group where m is 2 to 5;
- R^{2a}, R^{2b}: are each independently hydrogen, C₁-C₅ alkyl, or together form a-(CH₂)ₙ-group where n is 2 to 5, or C₂-C₆ alkenyl, or C₂-C₆ alkynyl;
- R³: is hydrogen,
- R^{4a}, R^{4b}: are each independently hydrogen, C₁-C₄ alkyl,
- R⁵: is hydrogen, C₁-C₄ alkyl, C(Hal)₃
- R⁶, R⁷: are each hydrogen, or together form an additional bond, or together form an epoxy function;
- G: is CH₂;
- D-E: is a group H₂C-CH₂, or
- D-E-G: together is a group H₂C-CH=CH
- W: is a group C(=X)R⁸, or is a bi- or tricyclic aromatic or heteroaromatic radical;
- X: is a group CR⁹R¹⁰;
- R⁸: is hydrogen, C₁-C₄ alkyl, halogen,
- R⁹, R¹⁰: are each independently hydrogen, C₁-C₄ alkyl, aryl, aralkyl,
- Z: is O
- A-Y: is a group O-C(=O), NR¹²-C(=O)
- R¹²: is hydrogen, or C₁-C₄ alkyl;
as a single stereoisomer or a mixture of different stereoisomers, and / or as a pharmaceutically acceptable salt thereof.

Still another aspect of the invention are combinations comprising epothilones of formula (V),
wherein
- R^{1a}, R^{1b} are: each independently hydrogen, C₁-C₂ alkyl, or together form a-(CH₂)ₘ-group where m is 2 to 5,
- R^{2a}, R^{2b} are: each independently hydrogen, C₁-C₅ alkyl, or together form a-(CH₂)ₙ-group where n is 2 to 5, or C₂-C₆ alkenyl, or C₂-C₆ alkynyl;
- R³: is hydrogen,
- R^{4a}, R^{4b} are: each independently hydrogen, C₁-C₂ alkyl,
- R⁵: is hydrogen or methyl or trifluormethyl,
- R⁶, R⁷: together form an additional bond, or together form an epoxy function;
- G: is CH₂;
- D-E: is a group H₂C-CH₂, or
- D-E-G: together is a group H₂C-CH=CH
- W: is a group C(=X)R⁸, or is thiazolyl, oxazolyl, pyridyl, N-oxo-pyridyl; benzothiazolyl, benzoxazolyl, benzimidazolyl, which are optionally substituted by C₁-C₃-alkyl, C₁-C₃-hydroxyalkyl, C₁-C₃-aminoalkyl, C₁-C₃-alkylsulfonyl
- X: is a group CR⁹R¹⁰;
- R⁸: is hydrogen, methyl, chloro, fluoro,
- R⁹, R¹⁰: are each independently hydrogen, C₁-C₄ alkyl, thiazolyl, oxazolyl, pyridyl, N-oxo-pyridyl, which are optionally substituted by C₁-C₃-alkyl, C₁-C₃-hydroxyalkyl, aralkyl,
- Z: is O
- A-Y: is a group O-C(=O), NR¹²-C(=O)
- R¹²: is hydrogen, or C₁-C₄ alkyl;
as a single stereoisomer or a mixture of different stereoisomers, and / or as a pharmaceutically acceptable salt thereof.

One embodiment of the invention are combinations comprising compounds of formula V wherein W is C(=X)R8 and X is CR⁹R¹⁰ or W is a mono-, bi- or tricyclic aromatic radical.

A further embodiment of the invention are combinations comprising compounds of formula V wherein W is a bi- or tricyclic aromatic radical.

A further embodiment of the invention are combinations comprising compounds of formula V wherein W is a bicyclic aromatic radical.

A further embodiment of the invention are combinations comprising compounds of formula V wherein W is a monocyclic aromatic radical.

An embodiment of the invention are combinations comprising compounds of general formula (V), wherein A-Y is O-C(=O); D-E is H₂C-CH₂; G is CH₂; Z is O; R^{1a}, R^{1b} are both C₁-C₁₀ alkyl or form together a -(CH₂)p- group where p is 2 to 3; R^{2a}, R^{2b} are each independently hydrogen, C₁-C₁₀ alkyl, C₂-C₁₀ alkenyl, or C₂-C₁₀ alkynyl; R³ is hydrogen; R^{4a}, R^{4b} are each independently hydrogen or C₁-C₁₀ alkyl; R⁵ is C₁-C₁₀ alkyl.

Another embodiment of the invention are combinations comprising compounds of the general formula (V), wherein R^{2a}, R^{2b} are each independently hydrogen, C₂-C₁₀ alkenyl or C₂-C₁₀ alkynyl; R⁶, R⁷ form an epoxy function or together form an additional bond.

Still a further aspect of the invention are combinations comprising compounds of formula (V) wherein W is a 2-Methylbenzothiazol-5-yl radical or a 2-Methylbenzoxazol-5-yl radical or a Quinoline-7-yl radical.

Another aspect of the invention are combinations comprising compounds of formula (V) wherein R^{2a}, R^{2b} are each independently hydrogen, C₂-C₁₀ alkenyl or C₂-C₁₀ alkynyl; R⁶, R⁷ form an epoxy function and W is a 2-Methylbenzothiazol-5-yl radical or a 2-Methylbenzoxazol-5-yl radical or a Quinoline-7-yl radical.

A special aspect of the invention are combinations comprising epothilones selected from the group consisting of
(4S,7R,8S,9S,13E/Z,16S)-4,8-dihydroxy-16-(2-methyl-benzoxazol-5-yl)-1-oxa-5,5,9,13-tetramethyl-7-(prop-2-en-1-yl)-cyclohexadec-13-ene-2,6-dione;
(1S/R,3S,7S,1 0R,11 R,12S,16R/S)-7,11-dihydroxy-10-(prop-2-en-1-yl)-3-(2-methyl-benzoxazol-5-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1S/R,3S,7S,10R,11S,12S,16R/S)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione;
(1S/R,3S,7S,10R,11R,12S,16R/S)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(2-methyl-benzothiazol-5-yl)-1-oxa-9,13-dimethyl-5,5-(1,3-trimethylen)-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(2-methyl-benzothiazol-5-yl)-12,16-dimethyl-8,8-(1,3-trimethylen)-4,17-dioxabicyclo[14.1.0]heptadecane-5,9-dione;
(4S,7R,8S,9S, 13E/Z, 16S)-4,8-dihydroxy-16-(2-methyl-benzothiazol-5-yl)-1-oxa-5,5,9,13-tetramethyl-7-(prop-2-in-1-yl)-cyclohexadec-13-ene-2,6-dione;
(1S/R,3S,7S,1 0R,11R,12S,16R/S)-7,11-dihydroxy-10-(prop-2-in-1-yl)-3-(2-methyl-benzothiazol-5-yl)-8,8,12,16-tetramethyl-4,17-dioxabicyclo[14.1.0]heptadecane-5,9-dione;
(4S,7R,8S,9S, 13E/Z, 16S)-4,8-dihydroxy-16-(quinolin-7-yl)-1-oxa-5,5,9,13-tetramethyl-7-(prop-2-en-1-yl)-cyclohexadec-13-ene-2,6-dione;
(1 S/R,3S,7S,10R,11R,12S,16R/S)-7,11-dihydroxy-10-(prop-2-en-1-yl)-3-(quinolin-7-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(1,2-dimethyl-1 H-benzoimidazol-5-yl)-1-oxa-5,5,9,13-tetramethyl-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(1,2-dimethyl-1 H-benzoimidazol-5-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione; (1S/R,3S,7S,10R,11S,12S,16R/S)-7,11-dihydroxy-10-(prop-2-en-1-yl)-3-(2-methyl-benzothiazol-5-yl)-8,8,12,16-tetramethyl-4-aza-17-oxabicyclo[14.1.0]heptadecane-5,9-dione, and (1S/R,3S,7S,10R,11R,12S,16R/S)-7,11-dihydroxy-10-(prop-2-en-1-yl)-3_(2-methyl-benzothiazol-5-yl)-8,8,12,16-tetramethyl-4-aza-17-oxabicyclo[14.1.0]heptadecane-5,9-dione,
(1S,3S(E),7S,10R,11S,12S,16R)-7,11-Dihydroxy-8,8,10,12,16-pentamethyl-3-[1-(2-methyl-1,3-thiazol-4-yl)prop-1-en-2-yl]-4,17-dioxabicyclo[14.1.0]heptadecane-5,9-dione ,
(1S,3S(E),7S,10R,11S,12S,16R)-7,11-Dihydroxy-8,8,10,12,16-pentamethy)-3-[1-(2-methyl-1,3-thiazol-4-yl)prop-1-en-2-yl]-17-oxa-4-azabicyclo[14.1.0]heptadecane-5,9-dione ,
(4S,7R,8S,9S,13Z,16S(E))-4,8-dihydroxy-5,5,7,9,13-pentamethyl-16-[1-(2-methyl-1,3-thiazol-4-yl)prop-1-en-2-yl]-oxacyclohexadec-13-ene-2,6-dione , (4S,7R,8S,9S,10E,13Z,16S(E))-4,8-dihydroxy-5,5,7,9,13-pentamethyl-16-[1-(2-methyl-1,3-thiazol-4-yl)prop-1-en-2-yl]-oxacyclohexadec-10,13-diene-2,6-dione , (4S,7R,8S,9S,10E,13Z,16S(E))-4,8-dihydroxy-5,5,7,9-tetramethyl-13-trifluormethyl-16-[1-(2-methyl-1,3-thiazol-4-yl)prop-1-en-2-yl]-oxacyclohexadec-10,13-diene-2,6-dione ,
(1 S,3S(E),7S,10R,11S,12S,16R)-7,11-Dihydroxy-8,8,10,12,16-pentamethyl-3-[1-(2-methylsulfanyl-1,3-thiazol-4-yl)prop-1-en-2-yl]-4,17-dioxabicyclo[14.1.0]heptadecane-5,9-dione
as a single stereoisomer or a mixture of different stereoisomers, and / or as a pharmaceutically acceptable salt thereof.

Most preferred for the combination of the present invention are
(4S,7R,8S,9S,13E/Z,16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1S/R,3S,7S,110R,11R,12S,16R/S)-7,11-d ihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione;
(1S/R,3S,7S,10R,11 S,12S,16R/S)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione or any stereoisomer thereof as a single stereoisomer or a mixture of different stereoisomers, and / or as a pharmaceutically acceptable salt thereof..
   One embodiment of the invention is any of the disclosed combinations comprising (a), a prostaglandin, (b) an epothilone and (c) a cyclooxygenase inhibitor with the proviso that the epothilone is not epothilone A, B, C or D.
   Another embodiment of the disclosed combinations is a combination comprising as epothilone (b) (1S,3S,7S,10R,11S,12S,16R)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione.
   or any stereoisomer or diastereoisomer thereof as a single stereoisomer or a mixture of different stereoisomers, and / or as a pharmaceutically acceptable salt thereof..
   One embodiment is the combination of (a) Iloprost [((E)-5-{(3aS,4R,5R,6aS)-5-Hydroxy-4-[(E)-(3S,4RS)-3-hydroxy)-4-methyl-1-octen-6-ynyl]perhydropentalen-2-ylidene}valeric acid] or Cicaprost [(5-{(E)-(1S,5S,6S,7R)-7-Hydroxy-6-[(3S,4S)-3-hydroxy-4-methyl-nona-1,6-diynyl]-bicyclo[3.3.0]oct-3-ylidene}-3-oxapentanoic acid)] or any pharmaceutically acceptable salt thereof and one of the epothilones(1S,3S,7S,10R,11S,12S,16R)-7,11-dihydroxy-10-(prop-2-en-1-yl)-3-(2-methyl-benzothiazol-5-yl)-8,8,12,10-tetramethyl-4,17-dioxabicyclo[14.1.0]heptadecane-5,9-dione or a pharmaceutically acceptable salt thereof Another embodiment is the combination of (a) Iloprost [((E)-5-{(3aS,4R,5R,6aS)-5-Hydroxy-4-[(E)-(3S,4RS)-3-hydroxy)-4-methyl-1-octen-6-ynyl]perhydropentalen-2-ylidene}valeric acid] or Cicaprost [(5-{(E)-(1 S,5S,6S,7R)-7-Hydroxy-6-[(3S,4S)-3-hydroxy-4-methyl-nona-1,6-diynyl]-bicyclo[3.3.0]oct-3-ylidene}-3-oxapentanoic acid)] or a pharmaceutically acceptable salt thereof and the epothilone (1S,3S,7S,10R,11S,12S,16R)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione or a pharmaceutically acceptable salt thereof and (c) acetylsalicylic acid or any of its derivatives, pharmaceutically acceptable salts and crystal modifications such as e.g. solvates, polymorphs thereof.
   One preferred embodiment is the combination of (a) Iloprost [((E)-5-{(3aS,4R,5R,6aS)-5-Hydroxy-4-[(E)-(3S,4RS)-3-hydroxy)-4-methyl-1-octen-6-ynyl]perhydropentalen-2-ylidene}valeric acid] or a pharmaceutically acceptable salt thereof and the epothilone (1S,3S,7S,10R,11S,12S,16R)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione or a pharmaceutically acceptable salt thereof
   Another preferred embodiment is the combination of (a) Iloprost [((E)-5-{(3aS,4R,5R,6aS)-5-Hydroxy-4-[(E)-(3S,4RS)-3-hydroxy)-4-methyl-1-octen-6-ynyl]perhydropentalen-2-ylidene}valeric acid] or a pharmaceutically acceptable salt thereof and the epothilone (1S,3S,7S,10R,11S,12S,16R)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione or a pharmaceutically acceptable salt thereof and (c) O-acetylsalicylic acid, or pharmaceutically acceptable salts or crystal modifications such as e.g. solvates, polymorphs thereof
   One aspect of the invention is the combination of (a) a prostaglandin and any subgroup thereof as defined in the description and (b) a tubulin/microtubule interfering agent and any subgroup thereof as defined in the description and (c) a cyclooxygenase inhibitor which is not a specific COX-2 inhibitor.
   One aspect of the invention is the combination of (a) a prostaglandin and any subgroup thereof as defined in the description and (b) a tubulin/microtubule interfering agent and any subgroup thereof as defined in the description and (c) a cyclooxygenase inhibitor which does not include compounds of formula VI 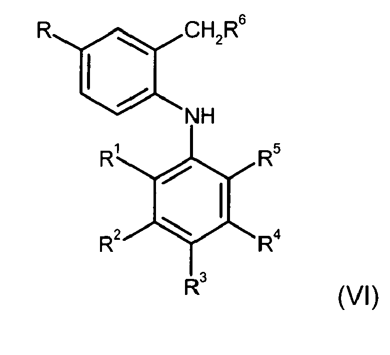 wherein
   R is methyl or ethyl
   R¹ is chloro or fluoro
   R² is hydrogen or fluoro
   R³ is hydrogen, fluoro, chloro, methyl, ethyl, methoxy, ethoxy, hydroxyl
   R⁴ is hydrogen or fluoro,
   R⁵ is chloro, fluoro, trifluoromethyl or methyl and
   R⁶ COOR⁷ or COOCH₂COOH.
   R⁷ is hydrogen, methyl, ethyl, 4-nitrooxybutyl.

One aspect of the invention is the combination of (a) prostaglandin and any subgroup thereof as defined in the description and (b) tubulin/microtubule interfering agent and any subgroup thereof as defined in the description and (c) a cyclooxygenase inhibitor which is an unspecific cyclooxygenase inhibitor. One aspect of the invention are combinations wherein the cyclooxygenase inhibitor is salicylic acid or any derivative, prodrug, pharmaceutically acceptable salt and crystal modification or solvate thereof.

Another aspect of the invention are combinations wherein the cyclooxygenase inhibitor is acetyl salicylic acid or any derivatives thereof, pharmaceutically acceptable salts and crystal modifications such as solvates, polymorphs thereof.

A further apect of the invention is a combination wherein the unspecific cyclooxygenase inhibitor (c) is acetyl salicyclic acid.

### Definitions

If somewere in the specification a carbon atom has only three of necessary four bonds actually defined and no further analogy can be found as to what should be added there a hydrogen should be regarded as intended.

The term "acyl" means -C(O)alkyl or -C(O)phenyl or -C(O)benzyl, the alkyl chain may be straight or branched and has up to 12 carbon atoms and the phenyl ring may optionally be substituted and the acyl residue is derived from the known carbonic acids such as, e.g. CH(O)OH, CH₃-COOH, CH₃-CH₂-COOH, CH₃-CH₂-CH₂-COOH, CH₃-CH₂-CH₂-CH₂-COOH, pivalic acid.

The term "alkyl" as used herein refers to straight-chain or branched-chain alkyl groups with 1-20 carbon atoms, e. g., methyl, ethyl, propyl, isopropyl, *n*-butyl, *t-*butyl, *n*-pentyl, isopentyl, neopentyl, heptyl, hexyl and decyl. Alkyl groups can be perfluorated or substituted by one to five substituents selected from the group consisting of halogen atoms, hydroxyl groups, C₁-C₄ alkoxy groups, or C₆-C₁₂ aryl groups (which can be substituted by one to three halogen atoms). If the term alkyl alone or in combination with other terms is used without giving any hint to the length of the chain the chain is meant to have at least one to five carbon atoms and the chain may be straight or branched and can optionally be substituted by hydroxy, halogen, methoxy.
The C₁-C₇ alkyl group of R² in the definition of prostaglandins may in addition be saturated or unsaturated.

The term "alkenyl" as used herein refers to straight or branched alkenyl groups, e.g. Vinyl-, Allyl-, Homoallyl-, (*E*)-But-2-enyl-, (Z)-But-2-enyl-, (*E*)-But-1-enyl-, (*Z*)-But-1-enyl-, Pent-4-enyl-, (*E*)-Pent-3-enyl-, (Z)-Pent-3-enyl-, (E)-Pent-2-enyl-, (Z)-Pent-2-enyl-, (*E*)-Pent-1-enyl-, (*Z*)-Pent-1-enyl-, 2-Methylvinyl-, 3-Methylbut-3-enyl-, 2-Methylbut-3-enyl-, (*E*)-2-Methylbut-2-enyl-, (Z)-2-Methylbut-2-enyl-, 3-Methylbut-2-enyl-Gruppe.

The term "alkynyl" as used herein refers to straight or branched alkynyl groups, e.g. C≡C, -CH₂-C≡CH, -C≡C-CH₃, -CH(CH₃)-C≡CH, -C≡C-CH₂(CH₃), -C(CH₃)₂-C≡CH, -C≡C-CH(CH₃)₂-, -CH(CH₃)-C≡C-CH₃, -CH₂-C≡C-CH₂(CH₃).

The term "alkoxy" as used herein refers to C₁-C₁₀-alkoxy groups, preferably C₁-C₅-alkoxy groups, that can be straight-chain or branched e.g. methoxy, ethoxy, n-propoxy, iso-propoxy, n-butoxy, iso-butoxy, tert-butoxy or n-pentoxy, 2,2-dimethylpropoxy, 2-methylbutoxy or 3-methylbutoxy group. A methoxy or ethoxy group is preferred.

The term "aryl" as used herein refer to a monocyclic aromatic carbocyclic e.g., phenyl, or heterocyclic ring moiety e.g. furyl, thienyl, pyridyl, pyrazolyl, pyrimidinyl, oxazolyl, pyridazinyl, pyrazinyl, thiazolyl, containing five to 14 ring atoms. Aryl groups can be substituted by one or more substituents selected from the group consisting of halogen, hydroxy, (C₁-C₁₀)alkoxy, -CO₂H, -CO₂(C₁-

C₁₀)alkyl, -NH₂, -NO₂, -N₃, -CN, (C₁-C₂₀)alkyl, (C₁-C₂₀)acyl, or (C₁-C₂₀)acyloxy. The heteroatoms can be oxidized, if as a result the aromatic character is not lost, e. g., a pyridine moiety can be oxidized to a pyridine N-oxide. Or the term "aryl" as used herein refers to a bi- and tricyclic aryl radical, which can be a substituted or unsubstituted carbocyclic or heterocyclic aryl radical with one or more heteroatoms, such as naphthyl, anthryl, benzoxazolyl, benzothiazolyl, benzimidazolyl, quinolyl, isoquinolyl, benzoxazinyl, benzofuranyl, indolyl, indazolyl, quinoxalinyl, tetrahydroisoquinolinyl, tetrahydroquinolinyl, thienopyridinyl, pyridopyridinyl, benzopyrazolyl, benzotriazolyl, or dihydroindolyl, which can be substituted in one or more places by halogen, OH, O-alkyl, CO₂H, CO₂-alkyl, -NH₂, -NO₂, -N₃, -CN, C₁-C₂₀-alkyl, C₁-C₂₀-acyl, or C₁-C₂₀-acyloxy groups, are suitable. The heteroatoms can be oxidized if as a result the aromatic character is not lost, such as, for example, the oxidation of a quinolyl to a quinolyl-N-oxide.

The term "aromatic or heteroaromatic radical" as used herein refers to mono-, bi- or tricyclic aromatic or heteroaromatic radicals whereby the bi- and tricyclic radicals are already defined under the term "aryl" and mono cyclic aromatic or heteroaromatic radicals can be selected from the group consisting of e.g. thienyl, furanyl, pyrrolyl, oxazolyl, thiazolyl, imidazolyl, pyrazolyl, isoxazolyl, isothiazolyl, oxadiazolyl, triazolyl, thiadiazolyl, phenyl, pyridyl, pyridazinyl, pyrimidinyl, pyrazinyl, triazinyl which also maybe substituted where possible by the substitutents mentioned for "aryl".

The term "aralkyl" as used herein refers to a group which can contain up to 14 atoms in the aryl ring (preferred five to ten) and one to eight carbon atoms in the alkyl chain (preferred one to four), e.g., benzyl, phenylethyl, naphthylmethyl, naphthylethyl, furylmethyl, thienylethyl, or pyridylpropyl. The rings can be substituted by one or more substituents selected from the group consisting of halogen, hydroxy, alkoxy, -CO₂H, -CO₂Alkyl, -NH₂, -NO₂, -N₃, -CN, C₁-C₂₀ alkyl, C₁-C₂₀ acyl, or C₁-C₂₀ acyloxy.

The term "aroyl" means (CO)aryl, e.g. benzoyl.

A "(C₄-C₇)cycloalkyl group" is e.g. cyclobutyl, cyclopentyl, cyclohexyl which may be optionally substituted by (C₁-C₅)alkyl, (C₁-C₅)alkoxy, halogen or hydroxy. If it contains an oxygen in the ring it is e.g. tetrahydrofuranyl.

The term "hal" or "halogen" means fluorine, chlorine, bromine or iodine, preferably in end products fluorine or chlorine, as a leaving group preferably iodine or bromine.

The term "5- or 6-membered heterocyclic ring having at least one nitrogen, oxygen or sulphur atom can be one of the following ring moieties being bound at any position chemically possible: piperidinyl-, morpholinyl-, thiomorpholinyl-, piperazinyl-, tetrahydrofuranyl-, tetrahydrothienyl-, imidazolidinyl- or a pyrrolidinyl ring system.

As substituents of the heterocylic group mentioned above can be selected from the group consisting of optionally substituted C₁-C₅-alkyl, hydroxy-, C₁-C₅-alkoxy-, NR⁴R⁵-, halogen, cyano-, COOR⁸-, CHO-. The substituents can be bound optionally also to the nitrogen atom if any; consequently also N-oxides are included in this definition.

The residue "CH₂N(alkyl, acyl)_{1,2}" means that the nitrogen can bear one or two same or different residues selected from alkyl or acyl and both terms, alkyl and acyl have the meaning as defined above.

The protecting groups "PG^{x,z}" can be alkyl- and/or aryl-substituted silyl moieties, such as tris(C₁-C₂₀ alkyl)silyl, bis(C₁-C₂₀ alkyl)-arylsilyl, (C₁-C₂₀ alkyl)-diarylsilyl, tris(aralkyl)-silyl, C₁-C₂₀ alkyl, C₂-C₂₀-alkenyl, C₄-C₇ cycloalkyl, which may contain an oxygen atom in the ring, aryl, aralkyl, C₁-C₂₀ acyl, aroyl, C₁-C₂₀ alkoxycarbonyl C₁-C₂₀ alkylsulfonyl or C₁-C₂₀ arylsulfonyl. Protecting groups which can be easily be removed from the molecule are preferred, e.g., methoxymethyl, methoxyethyl, ethoxyethyl, tetrahydropyranyl, tetrahydrofuranyl, trimethylsilyl, triethylsilyl, triisopropylsilyl, *t-*butyldimethylsilyl, t-butyl-diphenylsilyl, triphenylsilyl, tribenzylsilyl, benzyl, *p*-nitrobenzyl, *p*-methoxybenzyl, as well as alkylsulfonyl e.g. methylsulfonyl or arylsulfonyl e.g. p-tolylsulfonyl. As an alkoxycarbonyl radical, e.g., trichloroethyloxycarbonyl (Troc) is suitable. Preferred acyl groups are formyl, acetyl, propionyl, isopropionyl, trichloromethylcarbonyl pivaloyl, butyryl, or benzoyl, which all can be substituted by one or more amino and/or hydroxy moieties.

The "etherified or esterified hydroxy groups" result e.g. in (C₁-C₅)-alkoxy or (C₁-C₅)-alkoxy(C₁-C₅)alkyl groups e.g. MEM (Methylethoxymethyl), MOM (Methyloxymethyl), or cyclic ethers as e.g.THP (Tetrahydropyranylether), O(CO)(C₁-C₁₀)alkyl groups, O(CO)benzyl, O(CO)phenyl, with the used term alkyl as defined above.

As "amino protective groups PG", the radicals that are known to one skilled in the art are considered. For example, the Alloc, Boc, Z, benzyl, f-Moc, Troc, Stabase or benzostabase group can be mentioned.

The term "derivative" as used in combination with a class of compounds e.g. prostaglandins means structurally analogs of prostaglandins.

The term "carbacyclin" means any PGl₂-derivative having a carbon atom instead of the oxygen atom in the bicyclic ring skeleton. Thus carbacyclins are included in the term "PGl₂-derivatives or analogs". In addition the carbacyclin can be derivatized to yield carbacyclin derivatives which also are included in the term "PGl₂-derivatives or analogs"..

The term "tubulin/microtubule interfering" is used synonymously for microtubule stabilizing or tubulin stabilizing / microtubule depolymerizing.

The term "physiologically acceptable salts" of the components of the combination includes addition salts of mineral acids, carbonic acids, sulfonic acids, e.g. salts of hydrochloric acid, hydrobromic acid, sulfuric acid, phosphoric acid, nitric acid, methanesulfonic acid, ethanesulfonic acid, toluolsulfonic acid, benzenesulfonic acid, naphthalinesulfonic acid, acetic acid, trifluoroacetic acid, propionic acid, pivalic acid, lactic acid, tartaric acid, malic acid, citric acid, fumaric acid, maleic acid, succinic acid and benzoic acid.

In addition the term "physiologically acceptable salts" of the components of the combination includes salts of commonly suitable bases, e.g. salts of alkalimetall (e.g.. sodium- and potassium salts), alkaline earth salts (e.g. calcium- and magnesium salts) and ammonium salts, derivatized from NH₃ or organic amines with 1 to 16 carbon atoms, e.g. ethylamine, diethylamine, triethylamine, ethyldiisopropylamine, monoethanolamine, diethanolamine, triethanolamine, dicyclohexylamine, dimethylaminoethanol, prokaine, dibenzylamine, N-methylmorpholin, arginin, lysin, ethylendiamine and N-methylpiperidin.

"Solvates" in the sense of the invention are such forms of the compounds of the present combinations which build complexes by coordination of solvent molecules in a liquid or a solid phase. Hydrates are special forms of a solvate wherein water molecules are coordinated.

Stereoisomers or a mixture of different stereoisomers which means for compounds having one or more than one asymmetry center their enantiomers, diastereomers and racemates as well as E- and Z-Isomers for compounds containing double bonds

The kit of parts may contain ready-to-use formulations of one, two or all three components (a), (b), (c). It also may contain lyophylisates of one or more components which must be reconstituted before use. Its components may be administered simultanously, concurrently, separately or sequentially. The components of the combination may be administered independently of one another by oral, intravenious, topical or nasal application routes.

The combination of the present invention is useful for the treatment of a disease or condition associated with cell growth, division and/or proliferation, such as for treating malignant tumors in an individual in need thereof. As more specific applications, there can be mentioned, for example, the treatment of osteoporosis or osteoarthritis, the therapy of ovarian, stomach, colon, adeno-, breast, lung, head and neck carcinomas, malignant melanoma, acute lymphocytic, myelocytic leukaemia, bone-metastasis, brain tumours and brain metastases.

Thus in one aspect of the invention the combination can be used for the manufacture of a medicament for the treatment of a proliferative disease. In another aspect of the invention the combination can be used for the manufacture of a medicament for the treatment of a malignant tumor disease. In one aspect the combination is useful for the treatment of solid tumors.

In yet another aspect the combination is useful for the tretment of breast-, lung-, prostate-, ovarian cancers, melanoma, brain tumors and brain metastases.

In another aspect of the invention the combination is useful for the treatment of lung cancer including SCLC (Small Cell Lung Cancer) and NSCLC (Non Small Cell Lung Cancer).

In yet another aspect of the invention the combination is especially useful for the treatment of NSCLC (Non Small Cell Lung Cancer).

The combinations according to the invention are also suitable for treatment of chronic inflammatory diseases, such as, for example, psoriasis or arthritis. It follows that the combinations may be used for the preparation of a medicament for the treatment of the above-mentioned diseases.

Another further aspect of the invention relates to methods of treating diseases and conditions associated with hyper-proliferative processes in patients and more specific diseases as mentioned above comprising administering to the patient a therapeutically effective of the combination of the present invention.

A still further aspect of the invention is that the epothilone of the combination may be administered by intravenous injection or intravenous infusion over a period of about 5 minutes to 24 hours, preferentially 5 minutes to 3 hours or 30 minutes to 3 hours in a dose ranging from 5 mg/m² to 40 mg/m², preferentially from 12 mg/m² to 30 mg/m², especially 12mg/m², 16mg/m² or 22mg/m²

Alternatively the epothilone of the combination may be administered i.p. Another aspect of the invention is that the prostanoid of the invention may be administred orally together with the commonly suitable additives, by an intravascular route, topically or by inhalation. The recommended dose should lead to a systemical availability of 1-1000µg/kg/day depending on the actual formulation and use."

### Experimental section

The following equipment is used for conducting the experiments:

| | |
|---|---|
| Animals and strain | female, 50-days-old NMRI nude mice, body weight 20 g |
| Supplier | Taconics, Bomholdtgard; DK |
| Acclimatization | 4 days |
| Caging | Macrolon type III wire-mesh bottom under germ poor conditions |
| Feed type | Altromin R in metal hoppers |
| Drinking water | autoclaved tap water in water bottles (acidified to ph 2 with HCl) |
| Feeding and drinking time | ad libitum 24 hours per day |
| Room temperature | 24 °C |
| Relative humidity | 50-60 % |
| Light period | artificial; 10-hours dark/14 hours light rhythm (light 06.30 to 20.30 hours) |
| Identification | by cage labels |
| Tumor model | B52 human NSCLC cells were derived from a lung cancer patient and transplanted on nude mice |

### Description of the experiment

B-52 NSCL tumor pieces obtained from in vivo passage, were implanted s.c. in the inguinal region of female nude mice. Treatment was started when the tumors were approximately 25 mm² in size. Tumor area was determined by caliper measurements twice weekly. Treatment groups are shown in the table below.

For the purposes of the following experiment the epothilone derivative called "epothilone" in the table and experimental description below is (1S,3S,7S,10R,11 S,12S,16R)-7,11-d ihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

The following solutions/formulations are used for conducting the experiments:

| | **compound** | **dose/ concentration** | **vehicel %** | **amount/ dilution** |
|---|---|---|---|---|
| | epothilone | 8mg/kg 0,08ml/10gKG | Ampuwa | 1 vial + 4,6 ml Ampuwa comply with 10 mg/kg |
| | O-acetyl-salicylic acid | 60 mg/kg 0,1 ml/10gKG | HP-β-CD 30% | 600 mg/100 ml |
| | Iloprost | 0,2mg/kg 0,1ml/10gKG | NaCl 0.90% | solution.(Amp ulle=0,5 ml) 0,1mg/ml Verd.1:5 |
| | | | | |

### Part I:

The expectation for the experiment was:
It is known that prostaglandins selected from the subtypes PGI2, PGE1 or 6-Oxo-PGE1 induce vasodilation resulting in an improved blood flow. Therefore, treatment of tumor bearing mice with a prostaglandin before and after application of a potent anti-tumor agent epothilone should result in an improved circulation also in the necrotic parts of the solid tumors. By this, the antitumor agent should reach its target faster and more efficiently resulting in an improved antitumor effect.

### 1^{st} Experiment:

To demonstrate such an effect, human B52 NSCLC cells xenografted to nude mice were chosen as a model which is less sensitiv to chemotherapy.

### Description of study:

B-52 tumor pieces obtained from in vivo passage, were implanted s.c. in the inguinal region of female nude mice. Treatment was started when the tumors were approximately 25 mm² in size. Tumor area was determined by caliper measurements twice weekly. Tumor area was determined by caliper measurements.

6 groups were tested:

| **compound** | **Admin.** | **Regime** | **Dose** |
|---|---|---|---|
| 1. Untreated control, | i.v. | 1 x day29 | Vehicle |
| 2. epothilone | i.v. | 1 x day29 | 7 mg/kg |
| 3. epothilone | i.v. | 1 x day29 | 10 mg/kg |
| 4. Iloprost | i.v. | 1x/day, days 27-31 | 0.2 mg/kg |
| 5. epothilone + Iloprost | i.v. | Epothilone: 1 x day29 Iloprost: 1x/day, days 27-31 | 7 mg/kg // 0.2 mg/kg |
| 6. epothilone + Iloprost | i.v. | Epothilone: 1 x day29 Iloprost: 1x/day, days 27-31 | 10 mg/kg // 0.2 mg/kg |

### Results:

1. The stable IP-analog Iloprost alone had no significant effect on tumor growth.
2. The epothilone reduced tumor growth at the highest dose of 10 mg/kg which proved to be toxic in this model.
3. In contrast to the expectations described above the pretreatment with the stable PGl₂-analog Iloprost did not increase the anti-tumor effect of the epothilone.
4. Unexpectedly, the pretreatment with the stable PGl₂-analog Iloprost reduced the toxicity of the epothilone.

To test if the effect observed with the stable IP-analog Iloprost is mediated by endogenous metabolites of the arachidonic acid cascade, it was planned to inhibit these metabolites by pretreatment with the unspecific cyclooxygenase inhibitor O-acetyl salicylic acid and a COX inhibitor.

### 2^{nd} Experiment

The 2^{nd} Experiment was made to evaluate in a human B-52 NSCL cancer model, xenografted in nude mice the effect of:
- Iloprost and O-acetyl salicyclic acid alone,
- the combination of Iloprost and epothilone,
- the combination of O-acetyl salicyclic acid and epothilone,
- the combination of Iloprost and O-acetyl salicyclic acid as well as
- the combination of Iloprost, O-acetyl salicyclic acid and epothilone.

The study was designed to determine the response of a lung cancer model to a cytotoxic treatment regime with the epothilone derivative in combination with Iloprost, O-acetyl salicylic acid or the combination there'of. Basis for these additional combination are observations from experiment shown before, where Iloprost protected against the side effects the epothilone..
The tumor-inhibiting effect of, O-acetyl salicylic acid (ASS) and iloprost was investigated in the human B-52 NSCL cancer model, xenografted in nude mice. Description of study
B-52 tumor pieces obtained from in vivo passage, were implanted s.c. in the inguinal region of female nude mice. Treatment was started when the tumors were approximately 25 mm² in size. Tumor area was determined by caliper measurements twice weekly. Treatment groups were:

Tumor area was determined by caliper measurements.

| **compound** | **Admin.** | **Regime** | **Dose** |
|---|---|---|---|
| 1. Untreated control, | i.v. | At day24 | |
| 2. epothilone | i.v. | once day 24 | 8 mg/kg |
| 3. Iloprost | i.v. | Once each day from day 22-26 | 0.2 mg/kg |
| 4. O-Acetyl salicylic acid | p.o. | Once each day from day 19-29 | 60 mg/kg |
| 5. Iloprost + epothilone | i.v. | Iloprost: Once each day from day 22-26 | Iloprost: 0.2 mg/kg |
| | | epothilone: once day 24 | epothilone 8 mg/kg |
| 6. O-Acetyl salicylic acid + | p.o. | O-Acetyl salicylic acid : Once each day from day 19-29 | O-Acetyl salicylic acid : 60 mg/kg |
| epothilone | i.v. | once day 24 | 8 mg/kg |
| 7. Iloprost + | i.v. | Once each day from day 22-26; Once each day from day 19-29 | 0.2 mg/dakg |
| O-Acetyl salicylic acid + | p.o. | once day 24 | 60 mg/kg |
| epothilone | i.v. | | 8 mg/kg |
| 8. Iloprost + | i.v. | Once each day from day 22-26 | 0.2 mg/kg |
| O-Acetyl salicylic acid | p.o. | Once each day from day 19-29 | 60 mg/kg |

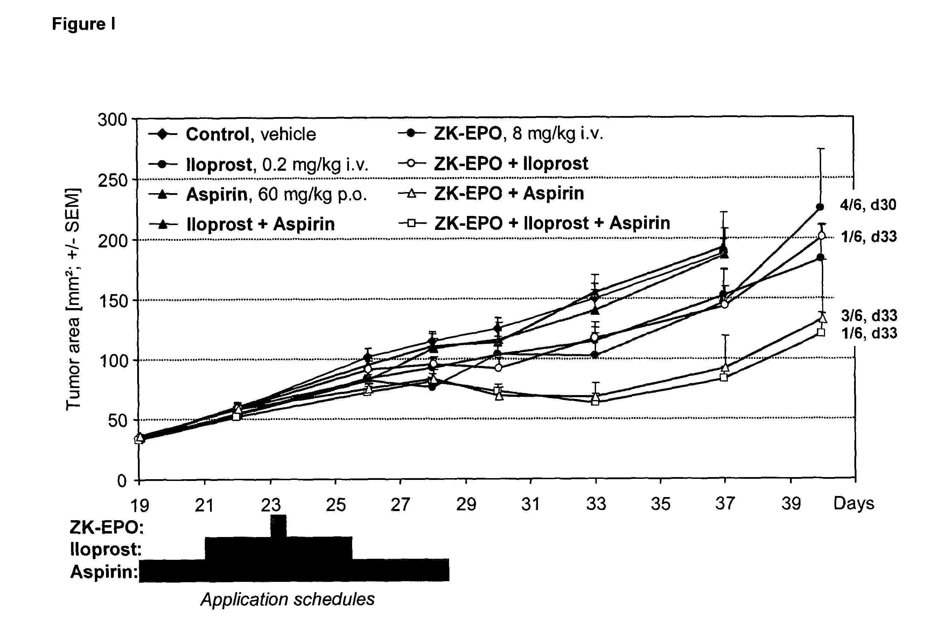

### Results: (shown in figure 1)

1. Treatment with Iloprost, O-acetyl salicylic acid or their combination without epothilone had no effect on tumor growth or bodyweight.
2. The epothilone slightly reduced tumor growth but showed already toxic effects.
3. The cotreatment of epothilone with O-acetyl salicylic acid resulted in a tumor regression and long term tumor growth inhibition. But this combination was still acompanied by severe toxicity (body weight loss> 15%, 3 mice died).
4. As before, the pretreatment with the stable PGl₂-analog Iloprost did not improve the anti-tumor effect of the epothilone but again significantly reduced its toxicity.
5. The treatment with O-acetyl salicylic acid Iloprost and epothilone resulted in a significant tumor growth inhibition (regression) compared to the epothilone alone, while the toxicity was also significantly reduced compared to the epothilone alone.

### Conclusions

The epothilone was effective in growth inhibiting the B-52 NSCL cancer model, whereas Iloprost had no effects.
The cotreatment of the epothilone with the unspecific COX-inhibitor O-acetyl salicylic acid resulted in a synergistically increased tumor growth inhibition.
The cotreatment of the epothilone with Iloprost resulted in an unexpected reduction of toxicity.
The addition of Iloprost to the combination of epothilone and O-acetyl salicylic acid unexpectedly reduced the toxicity without abating the antitumor effect.

## Claims

1. A combination comprising
(a) a prostaglandin or prostaglandine derivative, and
(b) a tubulin/microtubule interfering agent,
the compounds of the combination independently being in free form or in the form of their pharmaceutically acceptable salts.

2. A combination according to claim 1 further comprising
(c) a cyclooxygenase inhibitor in free form or in the form of its pharmaceutically acceptable salt.

3. A combination according to claims 1 or 2 wherein the prostaglandin or its derivative are selected from the group consisting of PGE₁, 6-Oxo-PGE₁, PGE₂, PGl₂ or analogs or derivatives thereof.

4. A combination according to claim 3 wherein the prostaglandin or prostaglandin derivative (a) is a PGl₂ derivative or analog thereof or a carbacyclin or a derivative or analog thereof.

5. A combination according to claim 4 wherein the carbacyclin derivative is a compound of formula (IV) 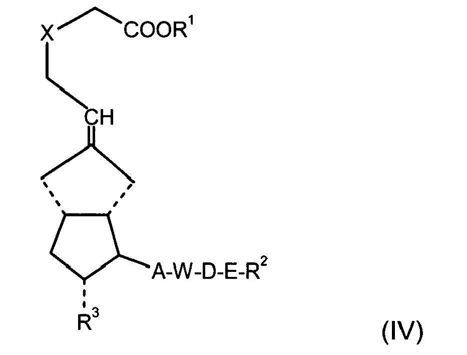 wherein
R¹ is hydrogen, a C₁-C₁₀-alkyl group being optionally substituted by halogen, phenyl, dimethylamino, diethylamino, C₁-C₅-alkoxy, a C₅-C₆-cycloalkyl group, a phenyl group being optionally substituted by halogen, C₁-C₄-alkoxy, hydroxyl or trifluoromethyl, or a 5 or 6 membered heterocyclic ring having at least one nitrogen, oxygen or sulphur atom,
A is a group selected from -CH₂-CH₂-, trans -CH=CH- or -C≡C-,
W is a hydroxy group which optionally may be etherified or esterified
or a group 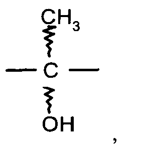 wherein the hydroxy group may optionally be etherified or esterified and the hydroxyl group may be in α- or β-position
D and E together form an additional bond or
D is a straight or branched C₁-C₁₀- alkylen group or an unsaturated C₂-C₁₀-alkenylene group which optionally may be substituted by fluorine atoms,
E is an oxygen atom or the group -C≡C-, or a direct bond and
R² is a C₁-C₇ alkyl group or a C₅-C₆-cycloalkyl group which both optionally may be substituted by halogen, an optionally substituted phenyl group, a C₅-C₆-cycloalkyl group, C₁-C₄-alkyl, chloromethyl, or a 5 or 6-membered heterocyclic ring having at least one nitrogen, oxygen or sulphur atom,
R³ is a hydroxyl group which optionally can be etherified or esterified and
X is a group selected from -CH₂- or -O-

6. A combination according to claim 4 wherein the prostaglandin or prostaglandin derivative is ((E)-5-{(3aS,4R,5R,6aS)-5-Hydroxy-4-[(E)-(3S,4RS)-3-hydroxy)-4-methyl-1-octen-6-ynyl]perhydropentalen-2-ylidene}valeric acid and (5-{(E)-(1S,5S,6S,7R)-7-Hydroxy-6-[(3S,4S)-3-hydroxy-4-methyl-nona-1,6-diynyl]-bicyclo[3.3.0]oct-3-ylidene}-3-oxapentanoic acid)or Misoprostol (9-Oxo-11α,16-dihydroxy-16-methyl-prost-13(E)-en-1-oic acid).

7. A combination according to claim 2 wherein the cylcooxygenase inhibitor (c) is an unspecific cyclooxygenase inhibitor.

8. A combination according to claim 7 wherein the cyclooxygenase inhibitor is salicylic acid or any derivative, prodrug, pharmaceutically acceptable salt and crystal modification or solvate thereof.

9. A combination according to claim 7 wherein the cyclooxygenase inhibitor is acetyl salicylic acid or any derivatives thereof, pharmaceutically acceptable salts and crystal modifications such as solvates, polymorphs thereof.

10. A combination according to any of the preceding claims wherein the microtubule inhibiting agent (b) is paclitaxel, docetaxel or an epothilone.

11. A combination according to any of the preceding claims wherein the microtubule inhibiting agent (b) is a compound of formula (V) 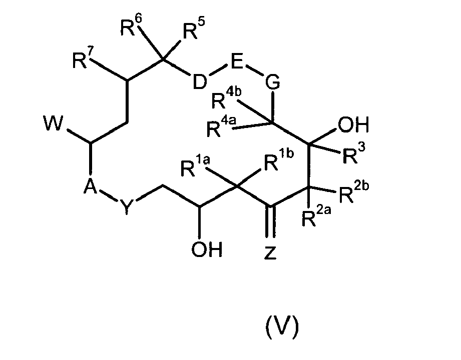 wherein:
R^{1a}, R^{1b} are each independently hydrogen, C₁-C₁₀ alkyl, aryl, aralkyl, or together form a -(CH₂)ₘ-group where m is 2 to 5;
R^{2a}, R^{2b} are each independently hydrogen, C₁-C₁₀ alkyl, aryl, aralkyl, or together form a -(CH₂)ₙ-group where n is 2 to 5, or are C₂-C₁₀ alkenyl or C₂-C₁₀ alkynyl;
R³ is hydrogen, C₁-C₁₀ alkyl, aryl, aralkyl;
R^{4a}, R^{4b} are each independently hydrogen, C₁-C₁₀ alkyl, aryl, aralkyl, or together form a -(CH₂)ₚ- group where p is 2 to 5;
R⁵ is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂N(alkyl, acyl)_{1,2}, or CH₂Hal, CHal₃;
R⁶, R⁷ are each hydrogen, or together form an additional bond, or together form an epoxy function;
G is O or CH₂;
D-E together is a group -H₂C-CH₂-, -HC=CH-, -C≡C-, -CH(OH)-CH(OH)-, -CH(OH)-CH₂-, -CH₂-CH(OH)-, -CH₂-O-, -O-CH₂- or CH-CH
whereby if G is O then D-E cannot be CH₂-O; or
D-E-G together is a group H₂C-CH=CH
W is a group C(=X)R⁸, or is an aromatic or heteroaromatic radical;
X is O or a group CR⁹R¹⁰;
R⁸ is hydrogen, C₁-C₁₀ alkyl, aryl, aralkyl, halogen, CN;
R⁹, R¹⁰ are each independently hydrogen, C₁-C₂₀ alkyl, aryl, aralkyl, or together with the methylene carbon form a 5- to 7-membered carbocyclic ring;
Z is O or H and the group OR¹¹;
R¹¹ is hydrogen or a protecting group PG^{z};
A-Y is a group O-C(=O), O-CH₂, CH₂-C(=O), NR¹²-C(=O), NR¹²-SO₂;
R¹² is hydrogen, or C₁-C₁₀ alkyl;
PG^{z} is C₁-C₂₀ alkyl, C₄-C₇ cycloalkyl, which may contain one or more oxygen atoms in the ring, aryl, aralkyl, C₁-C₂₀ acyl, aroyl, C₁-C₂₀ alkylsulfonyl, arylsulfonyl, tri(C₁-C₂₀ alkyl)silyl, di(C₁-C₂₀ alkyl) arylsilyl, (C₁-C₂₀ alkyl)diarylsilyl, or tri(aralkyl)silyl;
as a single stereoisomer or a mixture of different stereoisomers,and / or as a pharmaceutically acceptable salt thereof.

12. A combination of claim 11 wherein the epothilone is (1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione or a pharmaceutically acceptable salt thereof.

13. A combination of claim 2 wherein the compounds of the combination are
(a) ((E)-5-{(3aS,4R,5R,6aS)-5-Hydroxy-4-[(E)-(3S,4RS)-3-hydroxy)-4-methyl-1-octen-6-ynyl]perhydropentalen-2-ylidene}valeric acid] or a pharmaceutically acceptable salt thereof,
(b) (1S,3S,7S,10R,11S,12S,16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione or a pharmaceutically acceptable salt thereof and
(c) acetyl salicylic acid or any salts, crystal modification or polymorphs thereof.

14. Use of the combination of any of the preceding claims for the manufacture of a medicament for the treatment of a proliferative disease.

15. Use of claim 14, wherein the proliferative disease is a malignant tumor disease.

16. Use of claim 14, wherein the malignant tumor disease is lung cancer.

17. A Kit comprising a combination according to claim 1 or 2.

18. A kit according to claim 17 wherein optionally some components or all of them are in the form of a pharmaceutical formulation ready for use to be administered simultaneously, concurrently, separately or sequentially.
